# EUROPEAN PATENT SPECIFICATION

(11) **EP 2 008 585 B1**
(45) Date of publication and mention of the grant of the patent: **23.08.2017**
(21) Application number: 07013505.8
(22) Date of filing: 10.07.2007
(51) Int. Cl.: A61B 5/107, G01B 3/08, A61B 90/00

(54) **Measuring device for measuring a length of a through-hole in a bone**
Messgerät zur Messung der Länge eines Durchgangslochs in einem Knochen
Dispositif de mesure pour mesurer la longueur d'un trou traversant dans un os

(30) Priority: 28.06.2007 US 824590 P
(43) Date of publication of application: 31.12.2008
(73) Proprietor: Stryker European Holdings I, LLC, Kalamazoo, MI 49002 (US)
(72) Inventor: Prien, Ole, 24145 Kiel (DE); Giersch, Helge, 24235 Laboe (DE)
(74) Representative: Kühn, Ralph

(56) References cited:
- US-A- 3 738 355
- US-A- 4 850 354
- US-A- 5 013 318
- US-A1- 2005 066 535
- US-A1- 2006 207 118
- US-A1- 2007 088 366

## Description

### Field of the invention

The present invention relates to a measuring device and to a measuring kit for measuring a length of a through-hole in a bone.

### Technical background

It is known for example in osteosynthesis to provide a connection between an implant and a bone by elongated fixing elements such as screws. Therein, the fixing element may be inserted into the bone bicortically i.e. the fixing element extends into or through both cortex-parts at opposing surfaces of the bone. Therein, a through-hole through the bone having a diameter corresponding to the core diameter of the screw to be used for fixing the implant to the bone is usually drilled before inserting the screw. For this purpose, a hollow protection sleeve may be inserted through the patient's soft tissue surrounding the bone until it abuts to the bone. Then, a drill may be inserted through the protection sleeve and may drill the through-hole into the bone.

After pre-drilling the through-hole, a surgeon may have to select the screw having the correct length such that the screw extends into both of the opposing cortex regions of the bone while at the same time not protruding over the bone surface at the side of the bone opposite to the implant to be fixed on the bone.

In order to measure the thickness of the bone at the location of the pre-drilled through-hole, i.e. the length of the through-hole, a surgeon conventionally uses a measuring hook being simply a bar having a bent end section in order to having an L-shape. Therein, the bar has a diameter substantially smaller than the diameter of the through-hole. For measuring, the hook is inserted through the protection sleeve and through the through-hole and then the surgeon tries to hook the L-shaped hook-end into the opposite distal cortex of the bone. From the relative position of the hook-end attached to the distal cortex and the protection sleeve abutting the proximal cortex, the surgeon can determine the length of the through-hole and accordingly the correct length of the screw to be used.

However, using conventional measuring hooks, it may be difficult to fix the hook at the distal cortex as the hook may slip off the rim of the through-hole. Furthermore, there may be a risk to accidentally clamp some soft tissue between the distal hook-end and the cortex which, on the one hand, may falsify the measurement and, on the other hand, may injure the soft tissue.

US 2005/0066535 A1 discloses a device for length and depth measurements in surgery. US 3 738 355 discloses a bone gauge.

There may be a need for a measuring device or a measuring kit for measuring a length of a through-hole in a bone wherein the measuring device or the measuring kit can be fixed easily and reliably with respect to a through-hole in a bone or with respect to surface around such through-hole and where the risk of false measurement or injuring soft tissue is reduced.

### Summary of the invention

This need may be met by a measuring device and a bone thickness measuring kit according to the independent claims. Embodiments of the invention are described in the dependent claims.

According to a first aspect of the present invention, a measuring device for measuring a length of a through-hole in a bone is proposed, the measuring device comprising an elongated hollow sleeve having an opening at its distal extremity. Preferably, an outer cross-section of the sleeve is adapted to a cross-section of the through-hole. The measuring device further comprises a bar having a hook-end at its distal extremity. The bar is at least partly housed within the hollow sleeve and is slidable with respect to the sleeve along its longitudinal direction. Furthermore, the sleeve and the bar are adapted such that, slid to a distal position of the sleeve, the hook can be displaced such as to protrude through the opening of the sleeve and laterally over the lateral surface of the sleeve, and such that, slid to a proximal position of the sleeve, the bar does not protrude laterally over the outer cross-section of the sleeve.

It may be seen as a gist of the present invention that a measuring device is provided having a hollow sleeve and a bar slidably guided therein. The sleeve can be inserted into the through-hole the length of which has to be measured. As the outer cross-section of the sleeve is preferably adapted such as to substantially match the inner cross-section of the through-hole, the sleeve may be fixedly held within the through-hole while measuring the length of the through-hole. While inserting the sleeve into the through-hole, the bar may be located in the proximal position such as to not protrude laterally over the cross-section of the sleeve. After insertion of the sleeve into the through-hole, the bar can be brought to the distal position in which the hook-end protrudes laterally over the lateral surface of the sleeve. The measuring device can then be drawn back in the through-hole until the laterally protruding hook comes into abutment with the laterally surrounding surface of the bone, i.e. its cortex. In this position, the measuring device may be securely and fixedly held within the through-hole and there is no risk that the hook slips off the cortex as the sleeve is securely held within the through-hole and the hook-end laterally protrudes over the surface of the sleeve.

In the following, possible details and advantages of the present invention according to the first aspect and embodiments thereof will be explained.

Herein, the terms "distal" and "proximal" can be interpreted such that distal means away from a surgeon or from a position from which access to the through-hole can be obtained during an operation whereas proximal means closer to a surgeon.

Through-holes prepared in a bone are usually created by drilling such that they have a circular cross-section. Accordingly, the hollow sleeve may have a circular cross-section and may be e.g. in the form of a cylindrical tube. The outer cross-section or diameter of the sleeve may be selected such as to substantially correspond to the inner cross-section or diameter of the through-hole. In that way, the sleeve may be press-fit into the through-hole while measuring. Typical diameters for through-holes in bones are in the order of a few millimetres.

At its distal extremity and preferably at or close to its distal end, the sleeve has an opening. For example, the opening can extend in a lateral surface of the sleeve and can be provided as a longitudinal slit in the sleeve.

The bar to be fixed on a surface adjacent to the through-hole for measuring purposes and therefore having a hook-end at its distal extremity is usually, i.e. in the "proximal position" of the bar, completely contained within the hollow sleeve and does not protrude there from. The bar may have an outer cross-section corresponding to the inner cross-section of the hollow sleeve such that there is substantially no or little lateral play between the bar and the sleeve. However, the bar should be slidable along the longitudinal direction of the sleeve. By longitudinally sliding the bar into a specific position, named herein as "distal position" of the sleeve, the bar or its position can be modified such that its hook-end protrudes through the opening of the sleeve and laterally over the surface of the sleeve. By doing so, the local outer cross-section of the entire measuring device is increased at the position where the hook-end protrudes laterally.

For measuring the length of the through-hole, the distal end of the measuring device is slid through the through-hole with the bar in the non-protruding proximal position until the location of potentially increased cross-section is located distally (behind) of the through-hole. The bar will then be brought into the protruding distal position such that the cross-section is increased locally by the protruding hook-end. The measuring device can then be drawn into the proximal direction until the location of increased cross-section abuts to the cortex surrounding the through-hole. The length of the through-hole may then e.g. read from the scale formed on the outer surface of the sleeve.

According to an embodiment of the present invention the sleeve is substantially rigid and at least the distal extremity of the bar is substantially flexible, i.e. deflectable, such as to be bendable into a bent configuration to protrude over the lateral surface of the sleeve. In other words, the sleeve is adapted not to be bent while measuring the through-hole in the bone and accordingly the sleeve can act as a rigid guide to the bar housed at least partly therein. The bar, at least at its distal extremity, has a certain degree of flexibility. Due to this flexibility, the bar can be deflected by suitable means provided for example at the sleeve (to be described further below) such as to protrude over the lateral surface of the sleeve thereby increasing the local cross-section or diameter of the measuring device. Brought to the proximal position the bar may resume its original undeflected shape where its hook-end does not laterally protrude. For example, the sleeve may be a rigid metal tube and the bar may be a flexible metal wire housed within this tube.

According to a further embodiment, the hollow sleeve comprises a angled or tilted ramp at its inner side such that the ramp may force the distal extremity of the bar into the bent configuration when the bar is slid to the distal position of the sleeve. In other words, at its inner side, the hollow sleeve may have a region which is adapted to deviate the preferably flexible distal extremity of the bar when the bar is pushed distally within the sleeve. Accordingly, in its proximal position, the bar may be located away from the ramp and be housed completely within the hollow sleeve. In this position, the measuring device can be easily inserted into the through-hole to be measured. Then the bar can be pushed linearly to a distal position within the sleeve. Due to the ramp, the distal extremity having the hook-end thereon will be deviated from its linear direction and will be pushed laterally out of the opening at the distal extremity of the hollow sleeve. Thereby, the hook-end will protrude over the outer cross-section of the sleeve.

According to a further embodiment the sleeve extends longitudinally beyond the position of the hook-end being in its distal protruding position. In other words, the end of the sleeve is at a further distal position than the position of the hook-end when the bar is slid to the most distal position. Accordingly, when the measuring device is inserted into a patient's body, the distal end of the sleeve first pushes away any soft tissue which otherwise might interfere with the measurement. For example, when the measuring device is inserted through the through-hole to be measured, it will exit from the distal opening of the through-hole. As the sleeve extends distally beyond the hook-end, the sleeve will push away any soft tissue surrounding the through-hole such that there is no risk of clamping soft tissue when the laterally protruding hook-end is drawn into abutment with the distal cortex of the bone.

According to a further embodiment the distal extremity of the hollow sleeve is tapered. In other words, the outer cross-section of the sleeve reduces towards the distal end of the sleeve. For example, the distal end of the sleeve may be rounded or may have a tip in a cone-shape. The tapered distal extremity of the sleeve can be easily inserted into the through-hole in the bone the thickness of which is to be measured.

According to a further embodiment, the measuring device comprises a counter-piece being slidable with respect to the sleeve and being adapted for indication of a distance between the hook-end of the bar and a distal end of the counter-piece. In other words, additionally to the sleeve housing the bar, there is a counter-piece which may be slid along the longitudinal direction of the sleeve. Accordingly, for measuring the thickness of a bone at the location of a through-hole, the sleeve can be inserted into the through-hole and the hook-end of the bar can be protruded laterally from the sleeve and brought into abutment to the distal surface of the bone. Subsequently, the counter-piece can be slid along the sleeve in order to come into abutment with the proximal surface of the bone. The measuring device may then comprise a scale which indicates the distance between the hook-end of the bar and the distal end of the counter-piece thereby indicating the thickness of the bone positioned there between.

For example, both the sleeve and the counter-piece can be provided as cylindrical tubes, the sleeve having a slightly smaller diameter than the counter-piece such that the inner surface of the counter-piece can easily slide along the outer surface of the sleeve. In such embodiment, the counter-piece may also be used as hollow protection sleeve through which a drill may be guided when pre-drilling the through-hole. After drilling the through-hole, the counter-piece may remain in its position abuting the proximal cortex of the bone and the drill may be replaced by the sleeve-bar-combination of the measuring device for measuring the length of the through-hole.

According to a further embodiment, the measuring device further comprises a handle which can be attached to a proximal extremity of the sleeve and of the bar. Therein, the handle is adapted to displace the bar longitudinally along the sleeve upon activation of the handle. In other words, a handle can be provided which may be adapted to push and/or draw the bar linearly within the sleeve when the handle is activated in a specific way. When the handle is activated in order to push the bar to the distal position, this activation also induces protruding of the hook-end of the bar over the lateral surface of the sleeve. On the other side, when the handle is activated to draw the bar to the proximal position, the measuring device is brought into a state where the hook-end does not protrude such that the sleeve can be inserted into the through-hole.

Different kinds of possibilities of activation of the handle can be realized. According to a further embodiment, the handle is adapted to be activated by rotating a part of the handle. For example, the handle may comprise two parts, namely a static grip and a rotatable part which can be rotated around the longitudinal axis of the sleeve. Such two-piece handle may allow an easy handling of the measuring device. For example, the sleeve can be inserted into the through-hole to be measured and then the bar can be laterally protruded simply by turning the rotatable part of the handle.

Additionally, the handle can also be adapted such that the counter-piece can be attached to it. For example, the handle can be fixed to the counter-piece such that by pushing or withdrawing the handle, the counter-piece can be slid along the sleeve and can be brought into abutment with the proximal surface of the bone. In contrast to this linear movement of the handle for displacing the counter-piece, the handle can be rotated for activating the bar in order to bring it to the distal protruding position.

According to a further embodiment, the handle comprises an adapter such that the sleeve, the bar and/or the counter-piece can be releasably attached to the handle. For example the sleeve can be attached to the handle using a threading, a snap-in lock or a quick-release lock. Using such releasable attachment, the sleeve, the bar and/or the counter-piece can be easily removed from the handle e.g. for sterilization purposes. Furthermore, units including a sleeve and a matching bar and/or counter-pieces of different outer cross-sections can be attached to and released from the handle. Accordingly, the measuring device can be used for measuring through-holes of different diameters simply by replacing the sleeve by a sleeve of a suitable diameter.

According to a further aspect of the present invention, a bone thickness measuring kit is provided comprising a measuring device as described further above and further comprising a plurality of replacement sleeves having different outer cross-sections. Accordingly, for measuring a specific through-hole, the measuring device can be equipped with a replacement sleeve having a cross-section substantially corresponding to the cross-section of the through-hole.

The parts of the measuring kit and especially the replacement sleeves may be made from a material such as a metal like stainless steel which can be easily sterilized after each measuring process or may be provided as single-use pieces made for example from pre-sterilized inexpensive plastics.

It has to be noted that embodiments of the invention are described with reference to different subject-matters. In particular, some embodiments are described with reference to the measuring device whereas other features are described with reference to a method of using same. However, a person skilled in the art will gather from the above and the following description that, unless otherwise notified, in addition to any combination of features belonging to one type of subject-matter also any combination between features relating to the different subject-matters, in particular between features of the apparatus type claims and features of the method, is considered to be disclosed with this application.

The aspects defined above and further aspects, features and advantages of the present invention can be derived from the examples of embodiments described hereinafter.

The invention will be described in more detail hereinafter with reference to examples of embodiments but to which the invention is not limited.

### Brief description of the drawings

Fig. 1 shows a perspective schematic overview of a measuring device according to an embodiment of the present invention.
Fig. 2 shows a perspective view onto the distal extremity of a measuring device according to an embodiment of the present invention.
Fig. 3 shows a side view of the distal extremity of the measuring device shown in Fig. 2.
Fig. 4 shows a longitudinal cross-section of the measuring device shown in Fig. 2.
Fig. 5 shows an perspective explosion view from a first view point of a measuring device according to an embodiment of the present invention.
Fig. 6 shows an perspective explosion view from a different view point of the embodiment shown in Fig. 5.
Fig. 7 shows an enlarged side view of the region A indicated in Fig. 5.
Fig. 8 shows a cross section of the measuring device of Figs. 5 and 6.

In the figures, like reference signs designate like elements. Furthermore, it is to be noted that the figures are only schematically and not to scale.

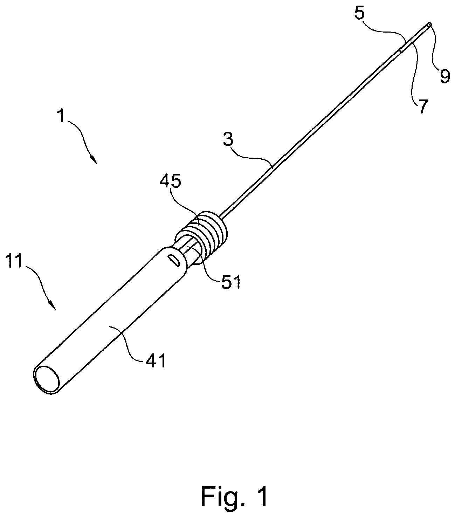

Fig. 1 schematically shows a measuring device 1 for measuring the length of a through-hole in a bone including an elongated hollow sleeve 3 with an opening 5 at its distal extremity and a bar 7 having a hook-end 9 at its distal extremity. The measuring device 1 further comprises a handle 11 including a rotatable grip 41, a static part 45 and a scale 15, wherein the rotatable grip 41 is adapted for displacing the bar 7 longitudinally along the sleeve 3.

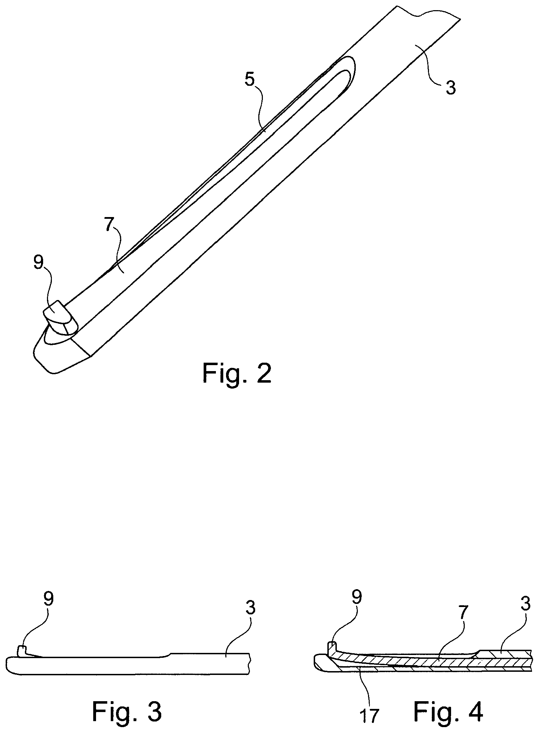

As can be seen from Figs. 2, 3 and 4, the distal extremity of the sleeve 3 comprises an opening 5 in the form of an elongated slit in the lateral surface of the sleeve 3. The opening 5 has a width corresponding to the width of the bar 7 housed within the sleeve 3 such that the bar 7 can come out of the sleeve 3 through the opening 5 at the distal part of the sleeve 3.

In a distal position where the bar 7 is pushed towards the distal extremity of the sleeve 3, the distal end of the bar 7 having the hook-end 9 formed thereon is pushed along a surface of a ramp 17 formed at the inner side at the most distal extremity of the sleeve 3. Thereby, the distal extremity of the bar having substantial mechanical flexibility is forced laterally out of the opening 5 (upwards in the drawings) such that the hook-end 9 protrudes over the lateral surface of the sleeve 3.

Brought to a proximal position (not shown in the figures), the bar 7 including the hook-end 9 can be completely housed within the sleeve 3 such that the hook-end 9 does not protrude over the lateral surface of the sleeve 3.

The distal end of the sleeve 3 is tapered and rounded in order to be able to easily insert the sleeve 3 into a pre-drilled through-hole within a bone without risking to damage the cortex of the bone.

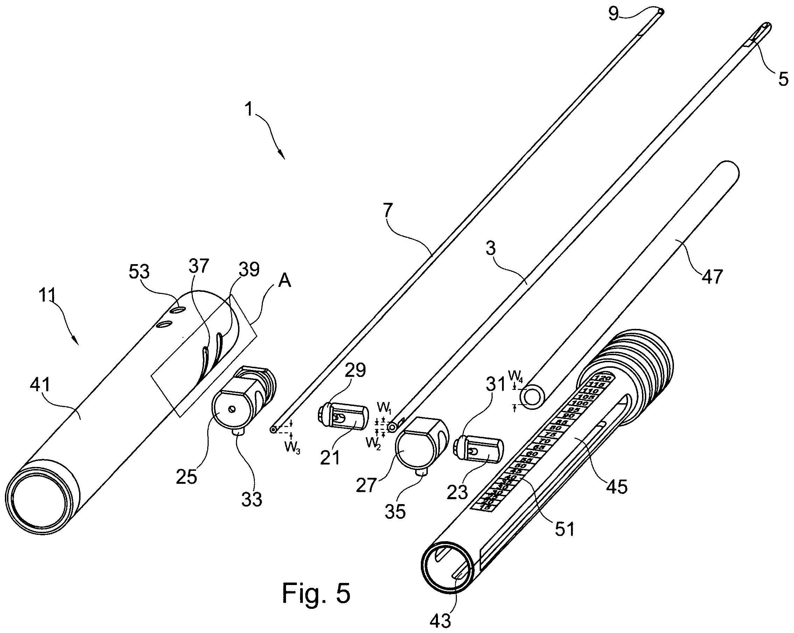

As shown in Figs. 5, 6 and 8, a measuring device 1 according to an embodiment of the present invention comprises the hollow cylindrical sleeve 3 and the cylindrical bar 7. The inner diameter w₂ of the hollow sleeve 3 is slightly larger (e.g. 2.1mm) than the outer diameter w₃ of the bar 7 (e.g. 2.0mm) such that the bar 7 can longitudinally slide within the sleeve 3. Both, the sleeve 3 and the bar 7 are held in holder pieces 21, 23. The holder pieces 21, 23 itself can be held in respective holders 25, 27 held by elastical O-rings 29, 31.

The holders 25, 27 to which the sleeve 3 and the bar 7 are attached have cams 33, 35 laterally protruding therefrom. When assembled, these cams 33, 35 can be guided within respective grooves 37, 39 formed in a grip 41 serving as rotatable part of the handle 11.

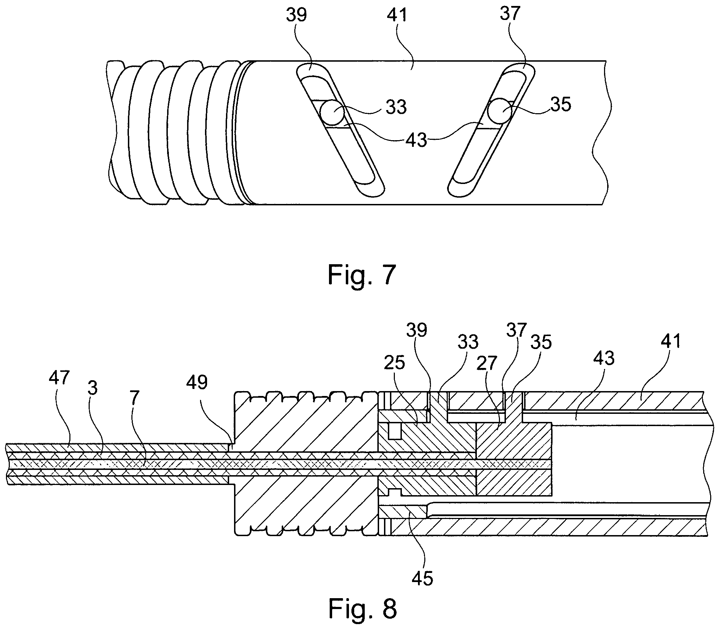

As can also be seen in Fig. 7 showing a side view of the area A indicated in Fig. 5, the two grooves 37, 39 are arranged at an angle with respect to each other. As the cams 33, 35 are guided within the grooves 37, 39 and as furthermore, the cams 33, 35 are guided in a linear groove 43 arranged in a static part 45 of the handle 11, by rotating the grip 41 the bar 7 can be linearly displaced with respect to the sleeve 3 along its longitudinal axis. In that way, the measuring device can be activated by rotating the grip 41 such that the bar 7 can either be brought to the distal position where its hook-end 9 protrudes through the opening 5 of the sleeve or to the proximal position, where the bar 7 is completely housed within the sleeve 3.

On the static part 45 of the handle 11, a counter-piece 47 can be attached by means of an adapter 49. The adapter 49 as well as the holder pieces 21, 23 can be adapted such that counter-pieces 47, bars 7 and/or sleeves 3 of different cross-sections or diameters can be attached to the handle 11. The counter-piece 47 can be a hollow cylinder having an inner diameter w₄ slightly larger than the outer diameter w₁ of the sleeve 3. Accordingly, the counter-piece 47 can be linearly slid along the sleeve 3. Furthermore, on the static part 45, a scale 51 is formed such that the distance between the distal end of the counter-piece 47 and the hook-end 9 of the bar 7 can be indicated and easily read through an opening 53 formed in the grip 41.

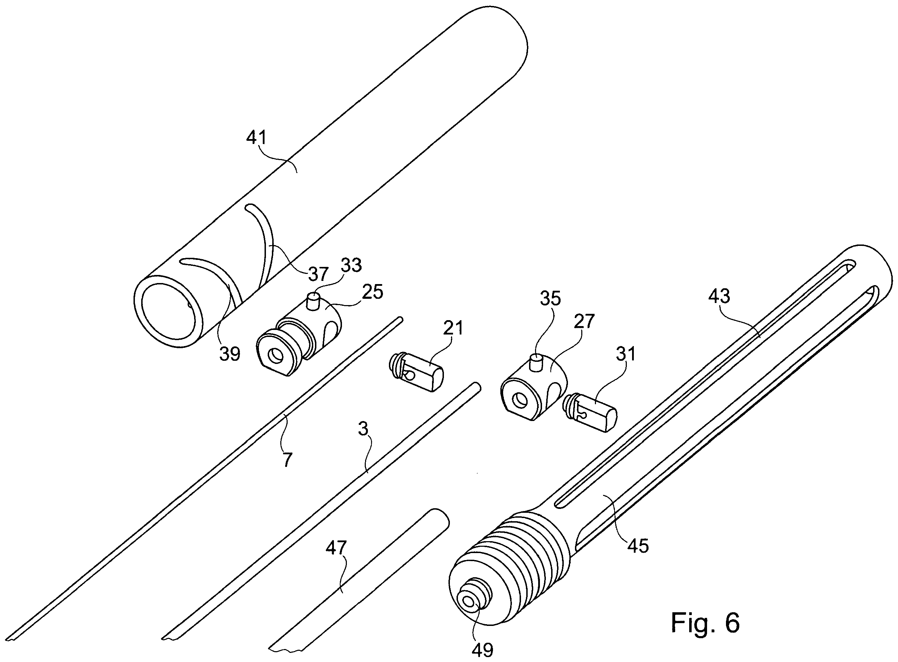

Fig. 8 shows a cross-section of the measuring device from which the interaction of the components shown in Figs. 5 and 6 can be derived. For clarity purposes, some minor details such as e.g. the holder pieces 21, 23 or features of the adapter 49 have been omitted in Fig. 8.

For measuring the length of a previously drilled through-hole within a bone of a patient, a surgeon first brings the bar 7 to its proximal position by rotating the grip 41 accordingly. In this withdrawn proximal position, the sleeve 3 can be easily inserted through the through-hole. Then, by turning the grip 41 accordingly, the surgeon brings the bar 7 to its distal position where the hook-end 9 protrudes from the lateral surface of the sleeve 3. The combination of sleeve 3 and bar 7 is then drawn back until the hook-end laterally abuts to the distal surface of the bone. Then, the handle 11 including the static part 45 with the counter-piece 47 attached thereto can be slid along the sleeve 3 until the distal end of the counter-piece 47 abuts to the proximal surface of the bone. The length of the through-hole is then indicated by the number of the scale 51 which can be read through the opening 53 of the handle 41.

It should be noted that the term "comprising" does not exclude other elements or steps and the "a" or "one" does not exclude a plurality. Also elements described in association with different embodiments and aspects may be combined. It should also be noted that reference signs in the claims shall not be construed as limiting the scope of the claims.

## Claims

1. A measuring device (1) for measuring a length of a through-hole in a bone, the measuring device comprising:
an elongated hollow sleeve (3) having a lateral opening (5) extending longitudinally on the lateral surface of the sleeve (3) at its distal extremity, a bar (7) comprising a hook-end lateral protrusion (9) formed on its distal extremity, said elongated hollow sleeve (3) and bar (7) being arranged such that the bar is at least partly housed within the hollow sleeve and is slidable with respect to the sleeve along its longitudinal direction, between a distal and a proximal position wherein the sleeve is substantially rigid and the distal extremity of the bar is substantially flexible such as to be bendable into a bent configuration for the hook-end lateral protrusion (9) to protrude over the lateral surface of the hollow sleeve (3), and
wherein the hollow sleeve comprises a angled ramp (17) at its inner side such that when the bar (7) is slid to the distal position the distal extremity of the bar is forced by the ramp (17) to deviate from a linear direction into a bent configuration with the hook-end lateral protrusion (9) being forced to protrude laterally through the lateral opening (5) and wherein, when the bar (7) is slid to the proximal position, the hook-end lateral protrusion (9) does not protrude laterally over the outer cross-section of the sleeve.

2. The measuring device according to claim 1,
wherein the sleeve extends longitudinally beyond the position of the hook-end when the bar is slid to its distal protruding position.

3. The measuring device according to one of the claims 1 and 2,
wherein a distal end of the sleeve is tapered.

4. The measuring device according to one of the claims 1 to 3,
further comprising a counter-sleeve (47) being slidable with respect to the sleeve and being adapted to indicate a distance between the hook-end of the bar and a distal end of the counter-sleeve.

5. The measuring device according to one of the claims 1 to 4,
further comprising a handle (11) attachable to a proximal extremity of the sleeve and of the bar, where the handle is adapted to displace the bar longitudinally along the sleeve upon activation of the handle.

6. The measuring device according to claim 5, wherein the handle is adapted to be activated by rotating a rotatable grip (41) of the handle.

7. The measuring device according to claim 5 or 6, wherein the handle comprises an adapter such that the sleeve can be releasably attached to the handle.

8. A bone thickness measuring kit comprising a measuring device according to one of claims 1 to 7 and a plurality of replacement sleeves having different outer cross sections.

## Patentansprüche

1. Messgerät (1) zum Messen einer Länge eines Durchgangslochs in einem Knochen, wobei das Messgerät Folgendes umfasst:
eine verlängerte hohle Hülse (3) mit einer seitlichen Öffnung (5), die sich der Länge nach auf der seitlichen Oberfläche der Hülse (3) an ihrer distalen Extremität erstreckt,
eine Stange (7), die einen auf ihrer distalen Extremität ausgebildeten seitlichen Überstand (9) eines Hakenendes umfasst, wobei die verlängerte hohle Hülse (3) und die Stange (7) derart angeordnet sind, dass die Stange wenigstens teilweise in der hohlen Hülse aufgenommen ist und bezogen auf die Hülse entlang ihrer Längsrichtung zwischen einer distalen und einer proximalen Position verschiebbar ist, wobei die Hülse im Wesentlichen fest ist und die distale Extremität der Stange im Wesentlichen derart flexibel ist, um in eine gebogene Konfiguration für den seitlichen Überstand (9) des Hakenendes biegbar zu sein, um über die seitliche Oberfläche der hohlen Hülse (3) hinauszuragen, und
wobei die hohle Hülse an ihrer Innenseite eine angewinkelte Rampe (17) derart umfasst, dass, wenn die Stange (7) zur distalen Position geschoben wird, die distale Extremität der Stange von der Rampe (17) gezwungen wird, von einer linearen Richtung in eine gebogene Konfiguration abzuweichen, wobei der seitliche Überstand (9) des Hakenendes dazu gezwungen wird, seitlich durch die seitliche Öffnung (5) hinauszuragen, und wobei, wenn die Stange (7) in die proximale Position geschoben wird, der seitliche Überstand (9) des Hakenendes nicht seitlich über den äußeren Querschnitt der Hülse hinausragt.

2. Messgerät nach Anspruch 1,
wobei die Hülse sich der Länge nach über die Position des Hakenendes hinaus erstreckt, wenn die Stange in ihre distale vorstehende Position geschoben wird.

3. Messgerät nach einem der Ansprüche 1 und 2,
wobei ein distales Ende der Hülse verjüngt ist.

4. Messgerät nach einem der Ansprüche 1 bis 3,
ferner umfassend eine Gegenhülse (47), die bezogen auf die Hülse verschiebbar ist und angepasst ist, einen Abstand zwischen dem Hakenende der Stange und einem distalen Ende der Gegenhülse anzugeben.

5. Messgerät nach einem der Ansprüche 1 bis 4,
ferner umfassend einen Halter (11), der an eine proximale Extremität der Hülse und der Stange angebracht werden kann, wobei der Halter angepasst ist, die Stange nach dem Aktivieren des Halters der Länge nach entlang der Hülse zu verschieben.

6. Messgerät nach Anspruch 5, wobei der Halter angepasst ist, durch Drehen eines drehbaren Griffs (41) des Halters aktiviert zu werden.

7. Messgerät nach Anspruch 5 oder 6, wobei der Halter einen Adapter umfasst, sodass die Hülse lösbar an dem Halter angebracht werden kann.

8. Knochendicken-Messset, umfassend ein Messgerät nach einem der Ansprüche 1 bis 7 und mehrere Ersatzhülsen mit verschiedenen äußeren Querschnitten.

## Revendications

1. Dispositif de mesure (1) destiné à mesurer une longueur d'un trou traversant dans un os, le dispositif de mesure comportant :
un manchon creux allongé (3) ayant une ouverture latérale (5) s'étendant longitudinalement sur la surface latérale du manchon (3) à son extrémité distale, une tige (7) comportant une saillie latérale d'extrémité de crochet (9) formée sur son extrémité distale, ledit manchon creux allongé (3) et ladite tige (7) étant agencés de telle sorte que la tige est au moins en partie reçue à l'intérieur du manchon creux et peut coulisser par rapport au manchon le long de sa direction longitudinale, entre une position distale et une position proximale, dans lequel le manchon est sensiblement rigide et l'extrémité distale de la tige est sensiblement souple de manière à pouvoir être pliée dans une configuration pliée pour la saillie latérale d'extrémité de crochet (9) pour faire saillie au-dessus de la surface latérale du manchon (3), et
dans lequel le manchon creux comporte une rampe inclinée (17) sur son côté intérieur de telle sorte que lorsque la tige (7) est coulissée jusqu'à la position distale, l'extrémité distale de la tige est forcée par la rampe (17) à dévier par rapport à une direction linéaire dans une configuration pliée, la saillie latérale d'extrémité de crochet (9) étant forcée à faire latéralement saillie à travers l'ouverture latérale (5) et dans lequel, lorsque la tige (7) est coulissée jusqu'à la position proximale, la saillie latérale d'extrémité de crochet (9) ne fait pas latéralement saillie au-dessus de la section transversale extérieure du manchon.

2. Dispositif de mesure selon la revendication 1, dans lequel le manchon s'étend longitudinalement au-delà de la position de l'extrémité de crochet lorsque la tige est coulissée jusqu'à sa position saillante distale.

3. Dispositif de mesure selon l'une des revendications 1 et 2, dans lequel une extrémité distale du manchon est amincie.

4. Dispositif de mesure selon l'une des revendications 1 à 3, comportant en outre un manchon complémentaire (47) pouvant coulisser par rapport au manchon et étant adapté pour indiquer une distance entre l'extrémité de crochet de la tige et une extrémité distale du manchon complémentaire.

5. Dispositif de mesure selon l'une des revendications 1 à 4, comportant en outre une poignée (11) pouvant être fixée à une extrémité proximale du manchon et de la tige, où la poignée est adaptée pour déplacer la tige longitudinalement le long du manchon lors d'une activation de la poignée.

6. Dispositif de mesure selon la revendication 5, dans lequel la poignée est adaptée pour être activée en faisant tourner un manche rotatif (41) de la poignée.

7. Dispositif de mesure selon la revendication 5 ou 6, dans lequel la poignée comporte un adaptateur de telle sorte que le manchon peut être fixé à la poignée de manière libérable.

8. Kit de mesure d'épaisseur d'os comportant un dispositif de mesure selon l'une des revendications 1 à 7 et une pluralité de manchons de remplacement ayant différentes sections transversales extérieures.
